# EUROPEAN PATENT SPECIFICATION

(11) **EP 2 552 485 B1**
(45) Date of publication and mention of the grant of the patent: **08.03.2017**
(21) Application number: 11721645.7
(22) Date of filing: 25.03.2011
(51) Int. Cl.: A61K 39/42, A61P 31/16, C07K 16/10

(54) **FULL-LENGTH IMMUNOGLOBULINS OF THE IGG ISOTYPE CAPABLE OF RECOGNIZING A HETEROSUBTYPE NEUTRALIZING EPITOPE ON THE HEMAGGLUTININ STEM REGION AND THEIR USE AS ANTI-INFLUENZA MEDICAMENT**
IMMUNOGLOBULINE MIT VOLLER LÄNGE VOM IGG-ISOTYP ZUR ERKENNUNG EINES HETEROSUBTYP-NEUTRALISIERENDEN EPITOPS AUF DER HÄMAGGLUTININ-STAMMREGION SOWIE DEREN VERWENDUNG ALS ANTIINFLUENZAMEDIKAMENT
IMMUNOGLOBULINES ENTIÈRE DE L'ISOTYPE IGG RECONNAISSANT UN ÉPITOPE NEUTRALISANT DE L'HÉTÉRO-SOUS-TYPE SUR LA RÉGION DE TIGE DE L'HÉMAGGLUTININE ET LEUR UTILISATION EN TANT QUE MÉDICAMENTS CONTRE LA GRIPPE

(30) Priority: 26.03.2010 IT TO20100237; 26.01.2011 IT TO20110067
(43) Date of publication of application: 06.02.2013
(73) Proprietor: Pomona Ricerca S.r.l., 10128 Torino (IT)
(72) Inventor: BURIONI, Roberto, I-20090 Segrate (Milano) (IT); CLEMENTI, Massimo, I-20129 Milano (IT)
(74) Representative: Comoglio, Elena
(86) International application number: PCT/IB2011/051284
(87) International publication number: WO 2011/117848

(56) References cited:
- WO-A1-2009/115972
- WO-A1-2009/144667
- WO-A1-2010/140114
- HENDERIKX PAULA ET AL: "A human immunoglobulin G1 antibody originating from an in vitro-selected Fab phage antibody binds avidly to tumor-associated MUC1 and is efficiently internalized", AMERICAN JOURNAL OF PATHOLOGY, vol. 160, no. 5, May 2002 (2002-05), pages 1597-1608, XP002644798, ISSN: 0002-9440
- BECK ALAIN ET AL: "Therapeutic antibodies and related products: choosing the right structure for success", M-S (MEDECINE SCIENCES), vol. 25, no. 12, December 2009 (2009-12), pages 1024-1032, XP8138357, ISSN: 0767-0974
- BURIONI R ET AL: "Monoclonal antibodies isolated from human B cells neutralize a broad range of H1 subtype influenza A viruses including swine-origin Influenza virus (S-OIV)", VIROLOGY, ACADEMIC PRESS,ORLANDO, US, vol. 399, no. 1, 30 March 2010 (2010-03-30), pages 144-152, XP026923963, ISSN: 0042-6822, DOI: DOI:10.1016/J.VIROL.2009.12.014 [retrieved on 2010-02-24]
- BURIONI ROBERTO ET AL: "Molecular cloning of the first human monoclonal antibodies neutralizing with high potency Swine-origin Influenza A pandemic virus (S-OIV)", NEW MICROBIOLOGICA, vol. 32, no. 4, October 2009 (2009-10), pages 319-324, XP002644800, ISSN: 1121-7138
- JOSTOCK T ET AL: "Rapid generation of functional human IgG antibodies derived from Fab-on-phage display libraries", JOURNAL OF IMMUNOLOGICAL METHODS, ELSEVIER SCIENCE PUBLISHERS B.V.,AMSTERDAM, NL, vol. 289, no. 1-2, 1 June 2004 (2004-06-01), pages 65-80, XP004520880, ISSN: 0022-1759, DOI: DOI:10.1016/J.JIM.2004.03.014
- D J SCHOFIELD ET AL: 'Variations in the neutralizing and haemagglutination-inhibiting activities of five influenza A virus-specific IgGs and their antibody fragments' JOURNAL OF GENERAL VIROLOGY, [Online] 01 October 1997, ENGLAND, pages 2431 - 2439, XP055185877 Retrieved from the Internet: <URL:http://vir.sgmjournals.org/cgi/content /abstract/78/10/2431>
- LAMARRE ALAIN ET AL: 'Protection from lethal coronavirus infection by immunoglobulin fragments' JOURNAL OF IMMUNOLOGY vol. 154, no. 8, 1995, pages 3975 - 3984, XP055185883 ISSN: 0022-1767

### SEQUENCE LISTING

<110> POMONA RICERCA SRL
<120> Whole immunoglobulin of the IgG isotype capable of recognizing an heterosubtype neutralizing epitope on the hemagglutinin stem region and their use as anti-influenza medicament
<130> PC1042EC
<160> 8
<170> PatentIn version 3.3
<210> 1
   <211> 122
   <212> PRT
   <213> Homo sapiens
<400> 1
   Leu Glu Glu Ser Gly Gly Gly Val Val Gln Pro Gly Arg Ser Leu Arg
   1               5                   10                  15
   Leu Ser Cys Ala Ala Ser Gly Phe Pro Phe Ser Ser Tyr Gly Met His
               20                  25                  30
   Trp Val Arg Gln Ala Pro Gly Lys Gly Leu Glu Trp Val Ala Gly Val
           35                  40                  45
   Ser Tyr Asp Gly Ser Tyr Lys Tyr Tyr Ala Asp Ser Val Lys Gly Arg
       50                  55                  60
   Phe Thr Ile Ser Arg Asp Ser Ser Lys Ser Thr Leu Tyr Leu Gln Met
   65                  70                  75                  80
   Asn Ser Leu Arg Pro Glu Asp Thr Ala Val Tyr Tyr Cys Ala Arg Pro
                   85                  90                  95
   Ser Ala Ile Phe Gly Ile Tyr Ile Ile Leu Asn Gly Leu Asp Val Trp
               100                 105                 110
   Gly Gln Gly Thr Thr Val Thr Val Ser Ser
           115                 120
<210> 2
   <211> 105
   <212> PRT
   <213> Homo sapiens
<400> 2
   Glu Leu Thr Gln Ser Pro Ser Ser Val Ser Ala Ser Val Gly Asp Arg
   1               5                   10                  15
   Val Thr Ile Thr Cys Arg Ala Thr Gln Gly Ile Ser Ser Trp Leu Ala
               20                  25                  30
   Trp Tyr Gln Gln Lys Pro Gly Lys Pro Pro Lys Leu Leu Ile Phe Gly
           35                  40                  45
   Ala Ser Ser Leu Gln Ser Gly Val Pro Ser Arg Phe Ser Gly Ser Gly
       50                  55                  60
   Ser Gly Thr Asp Phe Thr Leu Thr Ile Ser Ser Leu Gln Pro Glu Asp
   65                  70                  75                  80
   Phe Ala Thr Tyr Phe Cys Gln Gln Ala His Ser Phe Pro Leu Thr Phe
                   85                  90                  95
   Gly Gly Gly Thr Lys Val Glu Ile Lys
               100                 105
<210> 3
   <211> 366
   <212> DNA
   <213> Homo sapiens
<400> 3
   ctcgaggagt ctgggggagg cgtggtccag cctgggaggt ccctgagact ctcctgtgca     60
   gcctctggat tccccttcag tagttatggc atgcactggg tccgccaggc tccaggcaag    120
   gggctggagt gggtggcagg tgtttcatat gatggaagtt ataaatacta tgcggactcc    180
   gtcaagggcc gattcaccat ctccagagac agttccaaga gcactctata tctgcaaatg    240
   aacagcctga gacctgagga cacggctgtg tattactgtg cgagaccttc cgcgattttt    300
   ggaatataca ttattctaaa cggtttggac gtctggggcc aagggaccac ggtcaccgtc    360
   tcttca                                                               366
<210> 4
   <211> 315
   <212> DNA
   <213> Homo sapiens
<400> 4
   gagctcacgc agtctccatc ttccgtgtct gcatctgtag gagacagagt cactatcact     60
   tgtcgggcga ctcagggtat tagtagttgg ttagcctggt atcagcagaa accagggaaa    120
   ccacctaaac tcctgatttt tggtgcatct agtttgcaaa gtggggtccc atcaaggttc    180
   agcggcagtg gatctgggac agatttcact ctcaccatca gcagtctaca gcctgaagat    240
   tttgcaactt acttttgtca acaggctcac agtttcccgc tcactttcgg cggcgggacc    300
   aaggtggaga tcaaa                                                     315
<210> 5
   <211> 565
   <212> PRT
   <213> Influenza virus A (H1N1)
<400> 5
   Met Lys Ala Asn Leu Leu Val Leu Leu Cys Ala Leu Ala Ala Ala Asp
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Arg Leu Lys Gly Ile
       50                  55                  60
   Ala Pro Leu Gln Leu Gly Lys Cys Asn Ile Ala Gly Trp Leu Leu Gly
   65                  70                  75                  80
   Asn Pro Glu Cys Asp Pro Leu Leu Pro Val Arg Ser Trp Ser Tyr Ile
                   85                  90                  95
   Val Glu Thr Pro Asn Ser Glu Asn Gly Ile Cys Tyr Pro Gly Asp Phe
               100                 105                 110
   Ile Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Asn
       130                 135                 140
   Thr Asn Gly Val Thr Ala Ala Cys Ser His Glu Gly Lys Ser Ser Phe
   145                 150                 155                 160
   Tyr Arg Asn Leu Leu Trp Leu Thr Glu Lys Glu Gly Ser Tyr Pro Lys
                   165                 170                 175
   Leu Lys Asn Ser Tyr Val Asn Lys Lys Gly Lys Glu Val Leu Val Leu
               180                 185                 190
   Trp Gly Ile His His Pro Pro Asn Ser Lys Glu Gln Gln Asn Leu Tyr
           195                 200                 205
   Gln Asn Glu Asn Ala Tyr Val Ser Val Val Thr Ser Asn Tyr Asn Arg
       210                 215                 220
   Arg Phe Thr Pro Glu Ile Ala Glu Arg Pro Lys Val Arg Asp Gln Ala
   225                 230                 235                 240
   Gly Arg Met Asn Tyr Tyr Trp Thr Leu Leu Lys Pro Gly Asp Thr Ile
                   245                 250                 255
   Ile Phe Glu Ala Asn Gly Asn Leu Ile Ala Pro Met Tyr Ala Phe Ala
               260                 265                 270
   Leu Ser Arg Gly Phe Gly Ser Gly Ile Ile Thr Ser Asn Ala Ser Met
           275                 280                 285
   His Glu Cys Asn Thr Lys Cys Gln Thr Pro Leu Gly Ala Ile Asn Ser
       290                 295                 300
   Ser Leu Pro Tyr Gln Asn Ile His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Asn Pro Ser Ile Gln Ser Arg Gly Leu Phe Gly Ala Ile Ala Gly Phe
               340                 345                 350
   Ile Glu Gly Gly Trp Thr Gly Met Ile Asp Gly Trp Tyr Gly Tyr His
           355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Thr Val Ile Glu
   385                 390                 395                 400
   Lys Met Asn Ile Gln Phe Thr Ala Val Gly Lys Glu Phe Asn Lys Leu
                   405                 410                 415
   Glu Lys Arg Met Glu Asn Leu Asn Lys Lys Val Asp Asp Gly Phe Leu
               420                 425                 430
   Asp Ile Trp Thr Tyr Asn Ala Glu Leu Leu Val Leu Leu Glu Asn Glu
               435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465                 470                 475                 480
   Phe Glu Phe Tyr His Lys Cys Asp Asn Glu Cys Met Glu Ser Val Arg
                   485                 490                 495
   Asn Gly Thr Tyr Asp Tyr Pro Lys Tyr Ser Glu Glu Ser Lys Leu Asn
               500                 505                 510
   Arg Glu Lys Val Asp Gly Val Lys Leu Glu Ser Met Gly Ile Tyr Gln
           515                 520                 525
   Ile Leu Ala Ile Tyr Ser Thr Val Ala Ser Ser Leu Val Leu Leu Val
       530                 535                 540
   Ser Leu Gly Ala Ile Ser Phe Trp Met Cys Ser Asn Gly Ser Leu Gln
   545                 550                 555                 560
   Cys Arg Ile Cys Ile
                   565
<210> 6
   <211> 35
   <212> DNA
   <213> artificial
<220>
   <223> PCR primer
<400> 6
   caccatgaag gcaaacctac tggtcctgtt atgtg 35
<210> 7
   <211> 36
   <212> DNA
   <213> artificial
<220>
   <223> PCR primer
<400> 7
   tcagatgcat attctgcact gcaaagatcc attaga 36
<210> 8
   <211> 566
   <212> PRT
   <213> Influenza A virus (H3N2)
<400> 8
   Met Lys Thr Ile Ile Ala Leu Ser Tyr Ile Phe Cys Leu Ala Leu Gly
   1               5                   10                  15
   Gln Asp Leu Pro Gly Asn Asp Asn Ser Thr Ala Thr Leu Cys Leu Gly
               20                  25                  30
   His His Ala Val Pro Asn Gly Thr Leu Val Lys Thr Ile Thr Asp Asp
           35                  40                  45
   Gln Ile Glu Val Thr Asn Ala Thr Glu Leu Val Gln Ser Ser Ser Thr
       50                  55                  60
   Gly Lys Ile Cys Asn Asn Pro His Arg Ile Leu Asp Gly Ile Asp Cys
   65                  70                  75                  80
   Thr Leu Ile Asp Ala Leu Leu Gly Asp Pro His Cys Asp Val Phe Gln
                   85                  90                  95
   Asn Glu Thr Trp Asp Leu Phe Val Glu Arg Ser Lys Ala Phe Ser Asn
               100                 105                 110
   Cys Tyr Pro Tyr Asp Val Pro Asp Tyr Ala Ser Leu Arg Ser Leu Val
           115                 120                 125
   Ala Ser Ser Gly Thr Leu Glu Phe Ile Thr Glu Gly Phe Thr Trp Thr
       130                 135                 140
   Gly Val Thr Gln Asn Gly Gly Ser Asn Ala Cys Lys Arg Gly Pro Gly
   145                 150                 155                 160
   Ser Gly Phe Phe Ser Arg Leu Asn Trp Leu Thr Lys Ser Gly Ser Thr
                   165                 170                 175
   Tyr Pro Val Leu Asn Val Thr Met Pro Asn Asn Asp Asn Phe Asp Lys
               180                 185                 190
   Leu Tyr Ile Trp Gly Val His His Pro Ser Thr Asn Gln Glu Gln Thr
           195                 200                 205
   Ser Leu Tyr Val Gln Ala Ser Gly Arg Val Thr Val Ser Thr Arg Arg
       210                 215                 220
   Ser Gln Gln Thr Ile Ile Pro Asn Ile Gly Ser Arg Pro Trp Val Arg
   225                 230                 235                 240
   Gly Leu Ser Ser Arg Ile Ser Ile Tyr Trp Thr Ile Val Lys Pro Gly
                   245                 250                 255
   Asp Val Leu Val Ile Asn Ser Asn Gly Asn Leu Ile Ala Pro Arg Gly
               260                 265                 270
   Tyr Phe Lys Met Arg Thr Gly Lys Ser Ser Ile Met Arg Ser Asp Ala
           275                 280                 285
   Pro Ile Asp Thr Cys Ile Ser Glu Cys Ile Thr Pro Asn Gly Ser Ile
       290                 295                 300
   Pro Asn Asp Lys Pro Phe Gln Asn Val Asn Lys Ile Thr Tyr Gly Ala
   305                 310                 315                 320
   Cys Pro Lys Tyr Val Lys Gln Asn Thr Leu Lys Leu Ala Thr Gly Met
                   325                 330                 335
   Arg Asn Val Pro Glu Lys Gln Thr Arg Gly Leu Phe Gly Ala Ile Ala
               340                 345                 350
   Gly Phe Ile Glu Asn Gly Trp Glu Gly Met Ile Asp Gly Trp Tyr Gly
           355                 360                 365
   Phe Arg His Gln Asn Ser Glu Gly Thr Gly Gln Ala Ala Asp Leu Lys
       370                 375                 380
   Ser Thr Gln Ala Ala Ile Asp Gln Ile Asn Gly Lys Leu Asn Arg Val
   385                 390                 395                 400
   Ile Glu Lys Thr Asn Glu Lys Phe His Gln Ile Glu Lys Glu Phe Ser
                   405                 410                 415
   Glu Val Glu Gly Arg Ile Gln Asp Leu Glu Lys Tyr Val Glu Asp Thr
               420                 425                 430
   Lys Ile Asp Leu Trp Ser Tyr Asn Ala Glu Leu Leu Val Ala Leu Glu
           435                 440                 445
   Asn Gln His Thr Ile Asp Leu Thr Asp Ser Glu Met Asn Lys Leu Phe
       450                 455                 460
   Glu Lys Thr Arg Arg Gln Leu Arg Glu Asn Ala Glu Asp Met Gly Asn
   465                 470                 475                 480
   Gly Cys Phe Lys Ile Tyr His Lys Cys Asp Asn Ala Cys Ile Glu Ser
                   485                 490                 495
   Ile Arg Asn Gly Thr Tyr Asp His Asp Val Tyr Arg Asp Glu Ala Leu
               500                 505                 510
   Asn Asn Arg Phe Gln Ile Lys Gly Val Glu Leu Lys Ser Gly Tyr Lys
           515                 520                 525
   Asp Trp Ile Leu Trp Ile Ser Phe Ala Ile Ser Cys Phe Leu Leu Cys
       530                 535                 540
   Val Val Leu Leu Gly Phe Ile Met Trp Ala Cys Gln Arg Gly Asn Ile
   545                 550                 555                 560
   Arg Cys Asn Ile Cys Ile
                   565

## Description

The present invention generally falls within the immunology field. More specifically, the invention concerns full-length immunoglobulins capable of binding and neutralizing the influenza A virus.

Antibodies directed against the influenza A virus are known in the prior art.

The International Patent Application WO2009115972 describes monoclonal antibodies, preferably as Fab fragments, capable of binding a plurality of different influenza A virus subtypes (heterosubtypic immunity), such antibodies further being endowed with an important neutralization activity. The preferred antibody described in the above-mentioned International Patent Application is Fab 28 fragment, characterized in that it comprises a heavy chain variable region including the amino acid sequence SEQ ID NO:1 and a light chain variable region including the amino acid sequence SEQ ID NO:2. The respective encoding nucleotide sequences, that is SEQ ID NO:3 (heavy chain variable region) and SEQ ID NO:4 (light chain variable region) are also described in WO2009115972.

In the present specification, the antibody fragment Fab 28 is designated as "Fab PN-SIA28".

In the International Patent Application WO2009115972 the authors mention that the antibody subject of the invention may be alternatively provided as a full-length immunoglobulin as opposed to a Fab fragment. As known, human immunoglobulins are classified into five main classes: IgG, IgA, IgM, IgD, and IgE, which differ in the heavy chain type. However, in PCT/IB2009/051068, no specific immunoglobulin class is individually identified, and no suggestion regarding the heavy chain type is provided such as to allow the person of skill in the art to select a specific immunoglobulin class.

Moreover, in WO2009115972 no experimental data regarding the neutralizing effectiveness of PN-SIA28 antibody when used as a full-length immunoglobulin are provided.

Surprisingly, the present inventors have now found that a monoclonal antibody characterized by the amino acid sequences of the heavy chain variable region and the light chain variable region described in WO2009115972, specifically provided as a full-length IgG-class immunoglobulin, shows a dramatic increase in the neutralizing activity towards the influenza A virus, both in terms of power and in terms of range of action, compared to the corresponding Fab fragment.

As known, an IgG is made of a pair of light chains (L) and a pair of heavy chains (C). Each light chain contains two immunoglobulin domains, one variable (VL) and one constant (CL) domain. The heavy chains are of the γ type and each of them contains a variable immunoglobulin domain (VH or Vγ) and three constant domains (CH1/2/3). The γ chains are in turn classifiable into four different subtypes, designated as γ1, γ2, γ3, and γ4, respectively.

The monoclonal antibody as a full-length IgG, characterized by the amino acid sequences of the heavy chain variable region and the light chain variable region described in WO2009115972, is hereinafter designated as "IgG PN-SIA28".

Table 1 schematically shows the results obtained by the present inventors regarding the neutralizing activity of IgG PN-SIA28, expressed as comparisons.

There is evidence of a high neutralizing activity towards different strains of the H3N2 subtype, which instead is lower in the case of the corresponding Fab fragment.

Furthermore, even at extremely low concentrations, the full-length IgG exhibits a very powerful neutralizing activity towards different strains of the H1N1 subtype, higher than that of the corresponding Fab fragment against the same H1N1 strains. The present inventors have also performed preliminary experiments that allowed to assess the ability of IgG PN-SIA28 of also binding recombinant hemagglutinins belonging to subtypes H2, H5, and H9. The obtained results, which are described in detail in the experimental section, allow to believe that the neutralizing activity of IgG PN-SIA28 extends to at least subtypes H2, H5, and H9, which had not been previously described when referring to the corresponding Fab fragment.

Given the extraordinary neutralizing properties of IgG PN-SIA28, the present inventors have also performed particularly complex studies and experiments (illustrated in detail hereinafter) which allowed them to identify the hemagglutinin region constituting the epitope specifically recognized by the neutralizing antibody IgG PN-SIA28. The identification of the neutralization epitope recognized by IgG PN-SIA28 is particularly useful and important, making it possible to identify further human monoclonal antibodies directed against the same epitope and endowed with extraordinary neutralizing properties substantially comparable to those of IgG PN-SIA28.

Thus, one first object of the present invention is a human monoclonal antibody in the form of a full-length IgG, specifically directed against the influenza A virus hemagglutinin antigen and capable of binding and neutralizing a plurality of influenza A virus subtypes including at least the H1 subtype and the H3 subtype, characterized in that the antibody recognizes a conserved epitope located in the hemagglutinin stem region, said epitope comprising the amino acid residues His25, His45, Thr315 and Asn336 of the HA1 hemagglutinin polypeptide and the amino acid residues Thr358, Met360, Ile361 or Va1361, Asp362, Gly363, Trp364, Yhr384, Thr392, Val395, Glu400 of the HA2 hemagglutinin polypeptide, the numbering of the amino acid residues being based on the H1N1 hemagglutinin amino acid sequence available in the NCBI database under accession number EF467821.1 and designated in the sequence listing as SEQ ID NO: 5.

In a preferred embodiment, the human monoclonal antibody in the form of a full-length IgG which is the subject of the invention is capable of binding and neutralizing a plurality of influenza A virus subtypes including at least the H1 subtype, the H3 subtype and at least one among the H5, H2 and H9 subtypes. In another preferred embodiment, the human monoclonal antibody in the form of a full-length IgG which is the subject of the invention is capable of binding and neutralizing a plurality of influenza A virus subtypes including at least the H1 subtype, the H3 subtype and at least two among the H5, H2 and H9 subtypes.

In a further preferred embodiment, the human monoclonal antibody in the form of a full-length IgG which is the subject of the invention is capable of binding and neutralizing a plurality of influenza A virus subtypes including at least the H1 subtype, the H3 subtype, the H5 subtype, the H2 subtype and the H9 subtype.

In a further preferred embodiment, the human monoclonal antibody in the form of a full-length IgG which is the subject of the invention is IgG PN-SIA28, characterized in that it comprises a heavy chain variable region comprising the amino acid sequence SEQ ID NO:1 and a light chain variable region comprising the amino acid sequence SEQ ID NO:2.

The studies that led to the definition of the epitope recognized by the IgG PN-SIA28 antibody are described in detail in the experimental section that follows. Comparative studies carried out with reference to the epitope recognized by the prior art anti-influenza monoclonal antibodies C179, CR6261 and F10 are also described. Thanks to the comparative studies performed, the present inventors could prove that the above-mentioned prior art antibodies, which represent some of the most known and widespread anti-influenza antibodies, effectively recognize epitopes different from the epitope recognized by IgG PN-SIA28.

The following experimental section also illustrates the manufacture of IgG PN-SIA28 and the neutralization tests performed, with the relevant results.

The full-length IgG subject of the invention, preferably, but without limitation, the IgG PN-SIA28, is manufactured and used either in the free form or in a conjugated form with a carrier. A carrier is any molecule or chemical or biological entity designed to be conjugated to an antibody and modify the pharmacokinetic characteristics thereof, make it immunogenic or increase its immunogenicity. Non-limiting examples of carriers are proteins such as KLH ("keyhole limpet hemocyanin"), edestin, thyroglobulin, albumins such as bovine serum albumin (BSA) or human serum albumin (HSA), erythrocytes such as sheep red blood cells (SRBC), the tetanus anatoxin, the cholera anatoxin, poly-amino acids such as for instance poly(D-lysine:D-glutamic acid) and the like. In order to help the binding of the antibody to the carrier, the C-terminus or the N-terminus of the antibody can be modified, for instance by introduction of additional amino acid residues, for example one or more cysteine residues which are able to form disulphide bridges.

Due to its properties, illustrated in detail in the following experimental section with particular reference to IgG PN-SIA28, the full-length IgG subject of the invention is particularly suitable to be used in applications within the medical field, in particular for the manufacture of a medicament for the broad-spectrum prophylactic or therapeutic treatment of influenza A virus infections.

Thus, the use of the IgG of the invention, preferably, but without limitation, the IgG PN-SIA28, for the manufacture of a medicament for the prophylactic or therapeutic treatment of pathologies caused by influenza A virus infections, such as for example the influenza syndrome, falls within the scope of the invention.

The results of the neutralization tests performed and showed in the following experimental section induce to believe that the IgG PN-SIA28 is extremely effective in conferring a passive immunity against the influenza A virus in subjects to which such an IgG is administered, and that as a result it is particularly useful in the broad-spectrum prophylactic or therapeutic treatment of pathologies caused by influenza A virus infections, such as for example the influenza syndrome, particularly in a human being. Any other full-length IgG that recognizes the same hemagglutinin epitope which is recognized by IgG PN-SIA28 has neutralizing abilities substantially equal to those of IgG PN-SIA28.

Therefore, another object of the invention is a pharmaceutical composition comprising as the active ingredient an effective amount of the IgG of the invention, preferably, but without limitation, the IgG PN-SIA28, and a pharmaceutically acceptable carrier and/or diluent.

An effective amount of IgG is a quantity designed to fulfill a favorable effect in the subject to which the composition is administered, for instance to neutralize the influenza A virus by affecting a phase of its replication cycle.

In this context, the term "subject" refers to any animal host to which the composition is administered and performs its protective effect, preferably a mammal, more preferably a human being.

Non-limiting examples of pharmaceutically acceptable carriers or diluents useful in the pharmaceutical composition of the invention include stabilizers such as SPGA, carbohydrates (for example, sorbitol, mannitol, starch, saccharose, glucose, dextran), proteins such as albumin or casein, agents containing proteins such as bovine serum or non-fat milk, and buffers (for example phosphate buffer).

The IgG of the invention, preferably but without limitation the IgG PN-SIA28, may also be used advantageously as a diagnostic reagent in an *in vitro* method for detecting in a biological sample previously obtained from a patient (such as for example a serum, plasma, blood sample or any other suitable biological material, obtained from the patient, preferably a human being) anti-influenza A virus antibodies with a heterosubtype cross-neutralizing activity against the influenza A virus. These antibodies may be found in the biological sample obtained from the patient for instance as a result of a previous exposure to an influenza A virus, or because a monoclonal antibody of the invention had been previously administered to the patient for therapeutic or prophylactic or research purposes.

Therefore, an assay method for detecting, in a biological sample derived from a patient, the presence of anti-influenza A virus antibodies with a heterosubtype cross-neutralizing activity falls within the scope of the invention, the method comprising contacting said biological sample with the IgG of the invention, preferably but without limitation the IgG PN-SIA28, as a specific assay reagent.

The assay may be qualitative or quantitative. The detection and/or quantification of the anti-influenza A virus antibodies with a heterosubtype cross-neutralizing activity may be for example carried out by a competitive immunoassay, for instance a competitive ELISA, in which the ability of the biological sample previously obtained from the patient to displace the binding of the IgG of the present invention to hemagglutinin is assessed. The general features of the competitive immunoassays are generally known to the person of skill in the art and do not need a detailed description here.

Thus, a diagnostic kit comprising the IgG of the invention, preferably but without limitation the IgG PN-SIA28, as a specific reagent, also falls within the scope of the invention, said kit being in particular designed for the detection and/or quantification, in a biological sample previously obtained from a patient, of anti-influenza A virus antibodies with a heterosubtype cross-neutralizing activity against the influenza A virus.

Similarly, the IgG of the invention, preferably but without limitation the IgG PN-SIA28, may be used as a specific reagent in an assay method for detecting and/or quantifying, in a previously prepared immunogenic or vaccine composition, the epitope capable of evoking anti-influenza A virus antibodies with neutralizing properties identical or similar to those of the IgG of the invention, that is a heterosubtype cross-neutralizing activity against the influenza A virus, in the subject to which such a composition has been administered.

Such a method is predicted to be useful for the evaluation of any preparation that is to be used as a vaccine or immunogenic preparation, as the recognition by the monoclonal antibody of the invention is indicative of the presence, in the immunogenic preparation and/or vaccine, of one or more epitopes capable of stimulating the production of antibody clones capable of recognizing an advantageous epitope, such as for example an epitope capable of evoking a heterosubtypic immunity against the influenza A virus such as the one identified by the present inventors and described in detail in the present patent specification.

Finally, the IgG of the invention, preferably but without limitation the IgG PN-SIA28, may be used for the preparation of mimotopes, such as for example anti-idiotype antibodies, peptides, hemagglutinin truncated or artificial forms or others, endowed with the ability of evoking IgG PN-SIA28-like antibodies. Among these, the anti-idiotype antibodies are preferred. The anti-idiotype antibodies are antibodies specifically directed against the idiotype of the broad-spectrum neutralizing antibodies used for the manufacture thereof, and thus are able to mimic the key epitopes that they recognize. The manufacture of anti-idiotype antibodies is carried out by per se known methodologies that do not need further detailed explanations here. The paper published by the same inventors (Burioni et al. (2008) PLoS ONE 3(10):e3423, related to the manufacture of an anti-idiotype antibody capable of mimicking a critical epitope of the human immunodeficiency virus (HIV) gp120 is mentioned by way of example.

Thus, also mimotopes, preferably anti-idiotype antibodies, directed against an IgG of the invention, preferably but without limitation the IgG PN-SIA28, fall within the scope of the invention.

The following experimental section is provided by way of illustration.

### EXPERIMENTAL SECTION

### 1. PREPARATION OF IgG PN-SIA28 AND CHARACTERIZATION OF ITS NEUTRALIZING ABILITIES

IgG PN-SIA28, which represents a preferred embodiment of the invention, was generated by using baculoviruses. Sf-9 cell line was used for the transfection experiments and the subsequent production of high-titer virus stocks, whereas High Five cell line (H5) was used for the production of the recombinant proteins. Both of them are maintained under serumfree conditions, by using Sf-900 II SFM medium and Express-Five SFM (Gibco), respectively. The cells were incubated at 27°C and maintained in suspension at a concentration of about 10⁶ cells/ml.

### Preparation of the recombinant Baculovirus

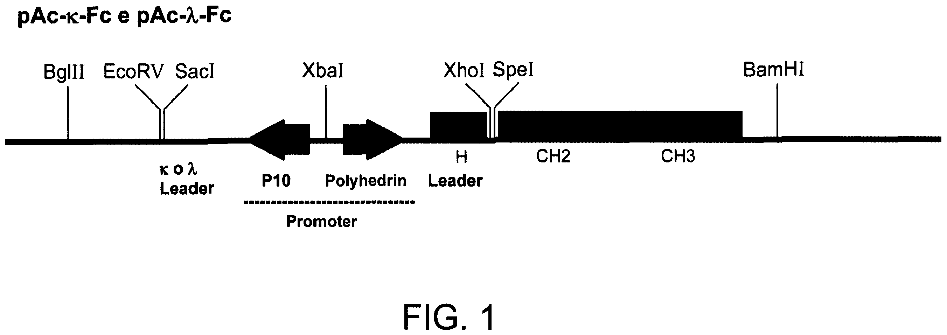

The generation of full-length IgGs starting from the Fab fragment was carried out by cloning the genes for the heavy and light chains into a transfer vector that contains regions homologous to the AcNPV baculovirus genome (genes for the promoters P10 and polyhedrin). The Baculovirus DNA (BD Biosciences Pharmingen) contains a lethal deletion, therefore it is not able to effect a productive infection once transfected into insect cells. When the baculovirus linearized genome is co-transfected into Sf-9 cells with the complementary transfer vector, the recombination between homologous regions results in the generation of a recombinant baculovirus, viable again and also able to express heterologous proteins. In particular, in order to generate the recombinant baculovirus that expresses the human full-length antibody, the transfer vector pAc-k-Fc (PROGEN) was used, which contains the Fc portion of the human IgG immunoglobulins and in which the genes for the light and heavy chains of the Fab PN-SIA28 have been cloned. The genes for the light chains were cloned into the transfer vector pAc-k-Fc by using the restriction sites *Sac*I*, EcoR*V*,* whereas the heavy chains by using the restriction sites *Xho*I*, Spe*I*.* Figure 1 shows a schematic representation of the transfer vector (Progen), wherein the cloning sites for the heavy and light chains are indicated.

The transfer vector, verified by digestion and sequencing tests, was thus co-transfected with 5µg of Baculovirus DNA (BD), following the protocol recommended by the manufacturer (BD Bioscences). The post-transfection medium was collected after approximately 6-7 days and analyzed for the presence of human immunoglobulins in an ELISA assay. This first supernatant was designated as "step 0" (SO).

In order to obtain a pure recombinant baculovirus population, a plaque assay was performed. Briefly, 2x10⁶ Sf-9 cells were seeded into a 6-well plate and serial dilutions (from 10⁴ to 10⁻⁷) of the supernatant S0 were prepared. 1 ml of each dilution was added to each well and after 1 hour at 27°C the inoculum was removed and a semi-solid medium (1% agarose solution) was added to each well. The plate was incubated at 27°C for approximately one week, that is until the appearance of lytic plaques detectable under a microscope. Several plaques were individually transferred into wells of a 24-well plate containing 10⁵ Sf-9 cells. After about one week, each supernatant was tested in an ELISA assay for the presence of IgGs secreted into the culture medium. Moreover, it was verified, again by an ELISA assay, that the antibody maintained its binding specificity towards the antigen. In particular, the recombinant IgG PN-SIA28 antibodies secreted into the culture medium were detected by a conventional capture ELISA assay, by using a polyclonal capable of binding human Fab fragments, as the capture reagent, and a horseradish peroxidase-conjugated polyclonal capable of binding the human Fc fragment, for the detection. In order to determine the specificity of the recombinant antibody, the ELISA assay was carried out by using, as the antigen, the influenza vaccine Inflexal V (season 2007-08), formerly recognized by the monoclonal as the Fab fragment.

### Amplification of the recombinant Baculovirus

Once obtained a pure population of recombinant baculoviruses by plaque assay, this was amplified to obtain high-titer virus stocks (approximately 10⁹ PFU/ml). Four *in vitro* amplification steps were performed, the first three (S1, S2, S3) in adherent Sf-9 cells, whereas the last step (S4) in suspended cells. S4 was then titrated in 96-well plates by the end-point dilution assay and the titer was calculated with the Reed-Muench formula.

### Expression of the recombinant antibody

In order to define the optimum expression conditions, H5 cells in suspension were infected at several Multiplicities of Infection (MOI 1, 5 and 10) and the medium was collected on different days (3, 4, 5, 6 days post-infection). The various aliquots were tested by Western Blot, both to establish the optimum infection conditions and to verify the integrity of the produced molecule. The produced recombinant immunoglobulins were detected with a horseradish peroxidase-conjugated polyclonal capable of binding the human Fc fragment. The assay was performed under denaturing and not reducing conditions (SDS 10%).

### Purification and quantification of recombinant PN-SIA28 IgGs

After having infected 1 L of H5 cells (MOI 5) at a concentration of 10⁶cells/ml, the cell viability was monitored daily by Trypan Blue staining. After about 4 days (approximately 70-80% mortality), the medium was collected, centrifuged and filtered with 0.2µm filters (Millipore). The recombinant antibody was then purified from the culture supernatant by affinity using the G protein. The antibody was eluted in 10 ml of elution buffer (0.1 M citric acid, pH 3) and concentrated by ultra-filtration through Amicon Ultra-15 (Millipore). The concentration and purity of the purified antibodies were determined by polyacrilamide gel (SDS-PAGE) and subsequent staining with Coomassie Blue, by referring to a BSA standard curve. Furthermore, the quantification of the recombinant protein was assessed by ELISA, by referring to a standard curve from human commercial IgGs.

### Micro-Neutralization Assay

MDCK cells (4x10⁴ per well) were seeded into a 96-well plate. Serial dilutions of Fab PN-SIA28 (20 µg/ml-0.078 µg/ml) and IgG PN-SIA28 (10 µg/ml-0.039 µg/ml) were incubated with 100 TCID₅₀ of each H1N1 or H3N2 virus. After 1 hour at 37°C, 100 µl of the virus-Fab or virus-IgG mixture at the different concentrations (for the infection control, the mixture is constituted by the virus alone) were added into each well. After 1 hour at 37°C, the 100 µl of the mixture were removed and 100µl of MEM TPCK-Trypsin were added into each well. After about 7 hours at 37°C, the cell monolayer was fixed and permeabilized with a cold ethanol solution for 10 minutes at room temperature. The cells were then incubated for 30 minutes at 37°C in a humidified chamber with an anti-influenza A monoclonal antibody (Argene) which was detected with a fluorescein isocyanate (FITC)-conjugated antibody. Finally, the cells were stained with the nuclear dye Hoechst 33342. The neutralizing activity of the antibodies was determined by calculating the decrease in the number of infected cells compared to the control virus, that is the virus not pre-incubated with the antibody of interest. In the experiments, a negative control was always included (virus + antibody not specific for the influenza virus), non-infected cells and a control for the toxicity of the antibodies used. The number of positive nuclei in each well was calculated by the automated GE Healthcare's IN Cell Analyzer System.

### RESULTS

### Generation of the full-length antibody through baculoviruses

The full-length immunoglobulins were obtained by cloning the genes for the Fab PN-SIA28 heavy and light chains into the transfer vector pAc-k-Fc (Progen) which contains the Fc portion.

After having co-transfected the Sf-9 cells with the vector pAc-k-Fc and the baculovirus linearized DNA, the inventors verified that the post-transfection culture medium contained human IgGs. Further, the IgGs secreted into the culture medium were tested for maintenance of the binding specificity. To this end, the post-transfection medium was tested by ELISA using, as the antigen, a polyclonal capable of binding human Fab fragments and the influenza vaccine Inflexal V (season 2007-08), respectively. In both cases, the monoclonal IgG PN-SIA28 was detected by a horseradish peroxidase-conjugated polyclonal capable of binding the human Fc fragment. 1% BSA was used as the negative control antigen. In parallel, the immunoenzimatic assay suitably adapted was also carried out using the Fab PN-SIA28. The ELISA results showed that the newly produced IgGs had been secreted into the culture medium after transfection, and had maintained their binding specificity, that is the ability to bind the influenza vaccine, as well as the Fab fragments derived therefrom.

The post-transfection medium was used for carrying out the plaque assay in order to obtain a pure population of recombinant baculoviruses. The supernatant from several individual plaques was tested by ELISA in order to assess the presence of IgGs secreted into the culture medium. Not all the supernatants resulted positive for the presence of immunoglobulins in the culture medium, and these were accordingly discarded.

After having obtained a high-titer virus stock (10⁸-10⁹ pfu/ml), the inventors monitored the expression of IgG PN-SIA28 by using different MOIs and different time-points. For the expression tests, the H5 cell line was used and the several medium aliquots collected on days 3, 4, 5 and 6 post-infection were tested by Western Blot, under denaturing and not reducing conditions. On the basis of the results obtained (data not shown), the inventors chose an MOI of 5 and to collect the medium on day 4 post-infection for the production of the recombinant protein in H5 cells.

### Characterization of the in vitro biological activity: micro-neutralization assay

Micro-neutralization assays in 96-well plates with several virus isolates belonging to the H1N1 and H3N2 subtypes were performed in order to assess the neutralizing activity of the antibodies. The experiments were carried out by using both the Fab PN-SIA28 and the IgG PN-SIA28. The results of the micro-neutralization assays are shown in the Table 1 below. The neutralizing ability of the antibodies is expressed as IC₅₀, that is the antibody concentration (expressed as µg/ml) capable of neutralizing the infection of the individual virus isolates by 50%:

**TABLE 1**

| | Fab 28 | IgG PN-SIA28 |
|---|---|---|
| H1N1 | | |
| A/Milan/UHSR1/2009 | 0.3 | 0.2 |
| A/WS/33 | 2.9 | 1.3 |
| A/Mal/302/54 | 0.8 | 0.64 |
| A/PR/8/34 | 2 | 1.2 |
| A/NC/20/99 | 7.0 | ND* |
| A/Swine/Parma/1/97 | 3.9 | 2 |
| | | |
| H3N2 | | |
| A/Hong Kong/8/68 | 20 | 0.8 |
| A/Aichi/2/68 | 20 | 0.6 |
| A/Victoria/3/75 | >20 | 1.2 |
| A/Port Chalmers/1/73 | 13.19 | 1.8 |
| A/Wisconsin/67/2005 | >20 | ND* |

| | | |
|---|---|---|
| * ND: experiment not done | | |

The assayed monoclonal resulted capable of neutralizing, both as a Fab fragment (Fab PN-SIA 28) and as a full-length immunoglobulin (IgG PN-SIA28), all the isolates tested belonging to the H1N1 subtype (reference ATCC strains and the 2009 pandemic H1N1 isolate). However, the recombinant proteins in the form of the full-length IgG show a neutralizing activity that is much higher than that of the Fab. The full-length immunoglobulin IgG PN-SIA28, in contrast with the Fab PN-SIA 28, also shows a significant neutralizing activity towards all the tested H3N2 subtype isolates. Therefore, on the whole, the full-length immunoglobulin IgG PN-SIA28 exhibits IC₅₀s definitely lower than those of the Fab.

### 2. IDENTIFICATION OF THE EPITOPE MATERIALS AND METHODS

### Selection and characterization of mutants capable of escaping the antibody response (antibody escape mutants) under Fab PN-SIA 28 selective pressure

The experiment was performed on 90% confluent MDCK cells grown in T25 flasks in MEM supplemented with 10% FBS (fetal calf serum). Fab PN-SIA 28 and e509 anti-E2/HCV were used, the latter used as a negative control (mock control), which were diluted in 1.5 ml of MEM supplemented with TPCK trypsin (2 µg/ml) in order to obtain final antibody concentrations of 2 pg/ml, 10 pg/ml and 20 µg/ml. 100 TCID₅₀ of A/PR/8/34 (ATCC no. VR-1469TM) were prepared in 1.5 ml of the same medium. The two solutions, containing Fabs and viruses, were mixed to obtain a final concentration of 1 µg/ml, 5 µg/ml and 10 µg/ml of the Fabs in a final volume of 3 ml. The mixtures were then incubated for 1 hour at 37°C. An infection positive control (virus without PN-SIA28) was also included, as well as non-infected cells. After two washes with sterile 1X PBS, 1 ml of each neutralization mixture was added to the flasks containing the MDCK cells, and an infection was carried out for 1 hour at 34°C in the presence of 5% CO₂. After the absorbance, the medium was removed and the monolayer was washed twice with sterile PBS. Three ml of MEM supplemented with trypsin (2 µg/ml) were added to the infection positive control and to the non-infected cells. Antibodies PN-SIA28 and e509 were added to the previously-treated infected cells, maintaining the concentrations used during the infection step. The cells were incubated at 34°C with 5% CO₂ and checked regularly for 48 hours for the presence of a cytopathic effect (CPE), comparing the infection positive control with the treated infected cells. The supernatant was then collected, centrifuged (2000 rcf for 10 minutes) and stored at -80°C. All the virus stocks were titrated and used for infecting new cell preparations, increasing where possible the Fab concentration. After 10 passages, the infection positive control and negative control (mock control) cells were compared with cells infected under PN-SIA28 selective pressure. When a strong cytopathic effect was evident in the positive and negative (mock) controls, the presence or absence of CPE was assessed in the PN-SIA28-treated infected cells. All the supernatants were collected, centrifuged, stored and used for sequencing the full-length DNA of the genomic fragment 4 of the influenza virus encoding the virus HA (hemagglutinin).

### Cloning and mutagenesis of HA

Hemagglutinin (HA) of A/PR/8/34 (H1N1) was amplified using the following PCR oligonucleotides:
APR834_s: 5'-CACCATGAAGGCAAACCTACTGGTCCTGTTATGTG-3' (SEQ ID NO:6);
APR834_as: 5'-TCAGATGCATATTCTGCACTGCAAAGATCCATTAGA-3' (SEQ ID NO:7).

The PCR products were cloned into the vector pcDNA 3.1D/V5-His-TOPO (Invitrogen).

Subsequently, mutants for H1N1 hemagglutinin (HA) and H3N2 HA were generated by using the Gene Tailor Site-Directed Mutagenesis System (Invitrogen). Twenty HA mutants for H1N1 and 4 HA mutants for H3N2 were generated in total.

### FACS binding assay

The binding activity of PN-SIA28 was assessed by using wild-type and mutant full-length HA proteins cloned as described above. In brief, human epithelial kidney (HEK) cells 293T were transfected with 4 µg of vector pcDNA 3.1D/V5-His-TOPO containing the HA nucleotide sequences. Following centrifugation and fixation with 4% paraformaldehyde, the transfected cells were incubated for 30 minutes at room temperature with IgG PN-SIA28 (10 µg/ml) or with a murine anti-H1 or anti-H3 (1 µg/ml). The cells were then washed and incubated for 30 minutes at room temperature with human or murine FITC-conjugated monoclonal anti-Fab antibodies. Thereafter, the cells were washed and analyzed by FACS. Non-transfected cells were also included in each experiment as a negative control. A monoclonal anti-murine H1 or H3 subtype antibody directed against a linear epitope was used to assess the transfection efficiency for each HA. The FACS analysis was carried out on cells wherein the signal obtained from those only stained with the secondary antibody was subtracted. The binding of PN-SIA28 to the different mutants was expressed as a binding percentage compared to the wild-type.

### Software

The following software were used for the analysis of the sequences: SeqScape (Applied Biosystems), ClustalX (Toby Gibson), Bio Edit (Tom Hall, Ibis Therapeutics), and Tree-view (GubuSoft). RasMol (Roger Sayle), Jmol (Jmol: a Java open-source viewer for 3D chemical structures. http://www.jmol.org/), Cn3D (United States National Library of Medicine, NLM), Pepitope server (Pepitope: mapping of epitopes from affinity-selected peptides. Bioinformatics 2007 23(23):3244-3246.), Mimox (BMC Bioinformatics 2006, 7:451 doi:10.1186/1471-2105-7-451) were used for visualizing and reproducing the molecules. Finally, GraphPad Prism was used for the analysis of the data and the graphical editing.

### RESULTS

### Characterization of the H1N1 virus hemagglutinin epitope recognized by the monoclonal antibody

### Selection and characterization of mutants capable of escaping the antibody response under Fab PN-SIA28 selective pressure

After having subjected the cells to the many steps described in the Methods, the cells infected with the wild type A/PR/8/34 H1N1 virus exhibited a strong cytopathic effect (CPE) both in the absence of antibody PN-SIA28 and in the presence of antibody e509 anti-E2/HCV used as a negative control. Cells infected with the wild type virus showed no evidence of CPE in the presence of PN-SIA28 at a concentration of 10 µg/ml. In contrast, cells infected with the selected variant virus showed a strong CPE in spite of the presence of PN-SIA28 at a concentration of 10 µg/ml. The sequencing of the generated variants capable of escaping the antibody response showed two different mutants, each of which having a single amino acid mutation in the HA2 subunit of the hemagglutinin stem region, compared to the amino acid sequence of the wild type virus. The two mutations are Ile361Thr and Asp362Gly (numbering referred to the linear A/PR/8/34 (H1N1) HA amino acid sequence, NCBI accession number EF467821.1, SEQ ID NO:5 in the sequence listing).

Either Isoleucine (I) or Valine (V) may be naturally present in position 361 in different strains of different subtypes. As described later, the I361V mutation does not affect the binding of PN-SIA28 to HA. No natural isolates exists which have Thr in position 361 or Gly in position 362.

### Identification of the HA region that binds mAb PN-SIA28

The antibody PN-SIA28 has a strong neutralizing activity against the influenza A virus. In particular, PN-SIA28 has a heterosubtype neutralizing activity against H1- (such as H1N1) and H3- (such as H3N2) subtype viruses, which belong to group 1 and group 2, respectively.

PN-SIA28, when tested in a hemagglutination inhibition assay, did not inhibit the virus-induced clustering of erythrocytes.

The Western blot analysis showed that only the immature form of HA (HA0) is recognized by PN-SIA28.

On the whole, the data obtained from the generation of antibody escape mutants, the hemagglutination inhibition assay and the Western blot analysis, suggest that the region recognized by the neutralizing antibodies does not lie within the HA globular region but in the stem region.

On the basis of these observations, HA molecules containing amino acid substitutions that may prevent the binding of the above-mentioned antibody were generated in order to characterize the epitope recognized by PN-SIA28.

In the following description, the amino acid residues are numbered on the basis of the linear A/PR/8/34 (H1N1) HA sequence, NCBI accession number EF467821.1, SEQ ID NO:5 in the sequence listing.

Firstly, the inventors considered that under the PN-SIA28 selective pressure, two different A/PR/8/34 (H1N1) HA2 antibody escape mutants were generated, in one of which the residue Ile361 was mutated into Thr and in the other the residue Asp362 was mutated into Gly. These two mutants are not recognized by PN-SIA28 anymore. On the basis of these results, HA mutants having an alanine in position 361 (substitution Ile361Ala) or an alanine/glycine in position 362 (substitution Asp362Ala, Asp362Gly) were generated and the ability of PN-SIA28 to bind the mutants was assessed by FACS analysis. PN-SIA28 could bind only weakly to the mutated HAs (Ile361Ala, Asp362Ala, Asp362Gly), demonstrating that these two residues are included in the epitope recognized by this antibody. It is important to note that the amino acid residues Ile361 and Asp362 are highly conserved in many hemagglutinin subtypes (H1, H2, H3, H4, H5, H6, H7, H8, H10, H14, H15).

A second group of mutants with mutations targeted to the possible region recognized by this IgG was designed in order to characterize in detail the epitope of PN-SIA28 HA Twenty mutants containing a substitution alanine in several amino acid positions, including Ile361Ala and Asp362Ala, were generated in total, see Table 2.

**TABLE 2**

| **HA1** | | **HA2** | |
|---|---|---|---|
| I. | His25 (His 18 F10 and CR6261) | I. | Trp357 |
| II. | His45 (His38 F10 and CR6261) | II. | Thr358 |
| III. | Thr315 (Thr318) | III. | Gly359 |
| IV. | Asn336 | IV. | Met360 |
| V. | Ile337 | V. | Ile361 (Val18 F10) |
| VI. | Pro338 | VI. | Asp362 (Asp 19 F10 and C 179) |
| | | VII. | Gly363 (Gly20 F10 and C179) |
| | | VIII. | Trp364 (Trp21 F10, CR6261, C179) |
| | | IX. | Thr384 (Thr41 F10 and CR6261) |
| | | X. | Ile388 (Ile45 F10 and CR6261) |
| | | XI. | Thr392 (Thr49 F10, CR6261 and C 179) |
| | | XII. | Val395 (Val 52 F10, CR6261 and C 179) |
| | | XIII. | Asn396 (Asn53 F10 and C179) |
| | | XIV. | Glu400 (Glu57 C179) |

In Table 2, the numbering is based upon the linear A/PR/8/34 (H1N1) HA sequence, NCBI accession number EF467821.1, SEQ ID NO:5 in the sequence listing. The numbering based on the original publications in which the antibodies F10, CR6261 and C 179 had been described is provided in brackets.

### Amino acid residues of the hemagglutinin region recognized by IgG PN-SIA28

The FACS analysis showed that the binding of the antibody PN-SIA28 was decreased in the following mutants: HA1-I, -II, -III, and -IV; HA2-II, -IV, -V, -VI, -VII, -VIII, -IX, -XI, XII and -XIV (cf. Table 2). These results indicate that the residues His25, His45, Thr315, Asn336, Thr358, Met360, Ile361, Asp362, Gly363, Trp364, Thr384, Thr392, Val395 and Glu400 are critical for the interaction between PN-SIA28 and HA. Thus, these residues are involved in the binding of the antibody to the stem region of HA. All the other mutations indicated in Table 2 did not result in a decrease in the binding of PN-SIA28 to HA.

An extra HA mutant was generated for position HA2-Ile361, wherein the original residue was changed into Valine. Within the same subtype, the natural sequence may contain in position 361 either an Isoleucine residue or a Valine residue. When the interaction of PN-SIA28 with the mutant HA Ile361Val was tested, no decrease in the interaction was observed. These results indicate that the antibody PN-SIA28 is capable of binding both of the virus variants, both the one with Isoleucine in position 361 and the one with Valine in the same position. This indicates that PN-SIA28 is able to bind and neutralize all the virus strains, irrespective of which of the two residues is present in position 361.

### Differences between the epitopes recognized by IgG PN-SIA28 and by the antibody C 179

The murine monoclonal antibody C 179, described in the state of the art, is able to bind a common epitope in the stem region of HA, shared by the HAs of subtypes H1, H2 and H5. The binding of the antibody C 179 to this region inhibits the fusion activity of HA and thus results in the neutralization of the virus. The epitope recognized by C 179 is composed of two different sites, one located in the HA1 subunit and defined by residues 318-322, and the other located in the HA2 subunit and defined by residues 47-58.

As the two sites are located close together in the center of the stem region of the HA molecule, C 179 appears to recognize these sites conformationally.

The mutant viruses capable of escaping the antibody response, containing HAs that carry one Thr to Lys substitution in position 318 of the HA1 subunit or one Val to Glu substitution in position 52 of the HA2 subunit, were not recognized and neutralized by C 179 anymore.

The epitope recognized by PN-SIA28 was compared to the one recognized by C 179.

The results from the FACS analysis showed that the amino acids His25, His45, Thr315, Thr358, Met360, Ile361, Asp362, Gly363, Trp364, Thr384 and Val395 (corresponding to mutants HA1-I, -II, and -III; HA2-II, -IV, -V, -VI, -VII, -VIII, -IX, and -XII, cf. Table 2) are important for the binding of PN-SIA28 to HA. These are not described as key residues for the binding of C 179 to HA. Moreover, Asn336, Thr392, Val395 and Glu400 (corresponding to mutants HA1-IV; HA2-XI, -XII and -XIV) are key residues for the interaction of HA with PN-SIA28, as well as for C 179. In addition, residue Asn396 (corresponding to the mutant HA2-XIII) is involved in the interaction between C 179 and HA, whereas it is not involved in the interaction between PN-SIA28 and HA.

In summary, the epitopes recognized by PN-SIA28 antibody and by C 179 antibody are different.

### Differences between the epitopes recognized by IgG PN-SIA28 and by the antibody CR6261

The antibody CR6261, described in the state of the art, has a heterosubtype neutralizing activity against the H1, H2, H5, H6, H8 and H9 subtypes of the influenza A virus. Crystallographic studies performed on the CR6261-A/South Carolina/1/1918 interaction showed that the antibody recognizes a highly conserved helical region located in the stem, proximal to the membrane on HA1 (residues 18, 38, 40, 42, 292) and HA2 (residues 21, 41, 45, 49, 52, 56). The antibody neutralizes the virus by blocking the conformational rearrangement associated with the fusion of the membrane.

The epitope recognized by PN-SIA28 was compared with the one recognized by CR6261.

Residues Thr315, Thr358, Met360, Ile361, Asp362, Gly363, Trp364 and Thr384 (corresponding to mutants HA1-III; HA2-II, -IV, -V, -VI, -VII, -VIII and -IX, cf. Table 2) are important for the binding of PN-SIA28 to HA but are not described as key residues for the binding of CR6261 to HA. Residues His25, His45 (corresponding to mutants HA1-I and-II, cf. Table 2), Thr392 and Val395 (corresponding to mutants HA2-XII and -XII, cf. Table 2) are key residues for the interaction of HA with PN-SIA28 and CR6261. Residue Ile388 (corresponding to the HA2-X mutant, cf. Table 2) does not appear to be important for the binding of PN-SIA28 to HA, whereas this residue was shown to be in direct contact with the antibody CR6261.

In summary, the epitopes recognized by PN-SIA28 and CR6261 are different.

### Differences between the epitopes recognized by IgG PN-SIA28 and by antibody F10

F10, an antibody described in the state of the art, is a high-affinity neutralizing antibody directed against hemagglutinin, extremely effective against highly pathogenic subtypes of the H5N1 and H1N1 viruses. This antibody inhibits the fusion process subsequent to attachment by recognizing a highly conserved epitope that is located in the hemagglutinin stem region. The crystal structure of F10 complexed with H5 from the A/Vietnam/1203/04 strain was determined. The epitope recognized by F10 includes a hydrophobic pocket formed by the HA2 fusion peptide flanked by HA1 residues on one side and by the HA2 αA helix on the other side. The HA1 region recognized by F10 includes residues 18 and 38, whereas the HA2 one includes residues 18-21, 41, 45, 49, 52, 53, 56. Studies effected with mutants showed that the residues Val52, Asn53 and Ile56 within the segment in HA2 αA, which performs important interactions with F10, strongly decrease or completely abolish the binding of the antibody.

The epitope recognized by PN-SIA28 was compared with the one recognized by F10.

Residues His25, His45, Ile361, Asp362, Gly363, Trp364, Thr384, Thr392 and Val395 (corresponding to mutants HA1-I and -II; HA2-V, -VI, -VII, -VIII, -IX, -XI and XII, cf. Table 2), important for the binding of PN-SIA28, are described as key residues for the binding of F10 to HA. Instead, residues Thr315, Asn336, Thr358, Met360 and Glu400 (corresponding to mutants HA1-III, -IV; HA2-II, -IV, and -XIV, Table 2) are key residues for the interaction of PN-SIA28 with HA but not for F10. In addition, Ile388 and Asn396 (corresponding to mutants HA2-X, -XIII, cf. Table 2) do not appear important for the binding of PN-SIA28 to HA, whereas this residue was shown to be in direct contact with F10.

In summary, the epitopes recognized by PN-SIA28 and F10 are different.

### Differences between IgG and Fab PN-SIA28 in the binding to HA

The HA original residues Thr315, Asn336, Pro338, Thr392, Glu400, when mutated into alanines, are only faintly recognized by IgG PN-SIA28. Instead, Fab PN-SIA28 is still able to bind strongly to these mutants. The present inventors assume that the differences in the binding characteristics may be caused by the steric hindrance due to the bigger size of the IgGs compared to the Fab fragments.

### Characterization of the epitope of the H3N2 virus hemagglutinin recognized by human monoclonal antibody PN-SIA28

A second group of HA mutants based upon H3N2 HA (A/Aichi/2/68) was created in order to assess if the epitope recognized by PN-SIA28 on H1N1 HA was also shared by H3N2 HA, see Table 3.

**TABLE 3**

| HA1 | HA2 |
|---|---|
| His34 (His25 H1N1) | Ile363 (Ile361 H1N1) |
| Asn54 (His45 H1N1) | Asp364 (Asp362 H1N1) |

The numbering is based upon the linear H3N2 HA sequence, NCBI accession number EF614251.1, SEQ ID NO:8 in the sequence listing.

### H3N2 HA amino acid residues recognized by PN-SIA28

The FACS analysis showed that the binding of PN-SIA28 to H3N2 HA decreased in mutants HA1-I and -II; HA2-I, -II, cf. Table 3. These results indicate that the residues His34, Asn54, Ile363 and Asp364 are critical for the interaction between PN-SIA28 and HA, thus they are a part of the epitope recognized by the antibody and are located in the stem region of HA.

The study with the mutants performed on H3N2 HA confirmed that the epitope recognized by PN-SIA28 is shared by the H1N1 and H3N2 hemagglutinins, despite the many amino acid differences found in the linear sequences of the two proteins.

The residues involved in the interaction of the antibody PN-SIA28 with HA are localized in highly conserved regions in most of the influenza A virus subtypes.

### 3. NEUTRALIZING ACTIVITY OF IgG PN-SIA28 AGAINST FURTHER INFLUENZA A VIRUS SUBTYPES

The neutralizing activity found for antibody IgG PN-SIA28 and the region that it recognizes allow for some considerations with regard to the neutralizing activity of this monoclonal also against other influenza virus subtypes. In this connection, it is useful to recall that up to now 16 HA subtypes have been distinguished, only three of which have been able to determine the pandemics documented in man (H1, H2 and H3), whereas other three of them (H5, H9 and H7) have been isolated sporadically from humans, but till now have not been able to cause a pandemic (Fouchier RA, Munster V, Wallensten A, et al. Characterization of a novel influenza A virus hemagglutinin subtype (H16) obtained from black-headed gulls. J Virol 2005;79(5):2814-22).

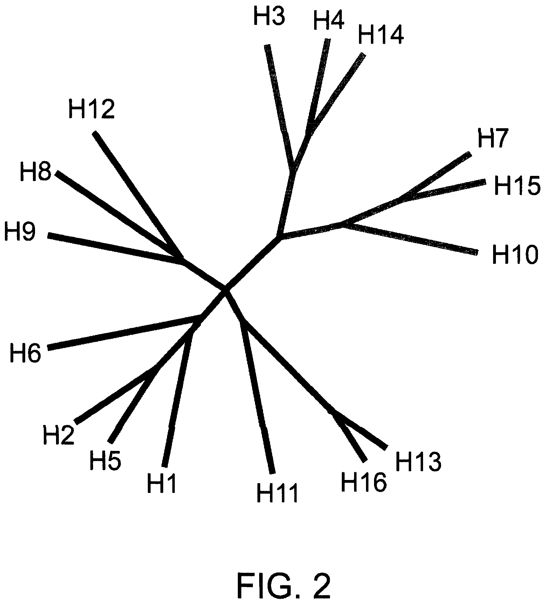

On the basis of the phylogenic distance between the 16 subtypes (from 40% to 60% homology), two phylogenic groups are typically distinguished: group 1 to which H1, H2, H5, H6, H8, H9, H11, H12, H 13 and H16 belong, and group 2 to which H3, H4, H7, H10, H 14, and H15 belong (Lambert LC, Fauci AS. Influenza vaccines for the future. N Engl J Med 2010;363(21):2036-44; Nabel GJ, Fauci AS. Induction of unnatural immunity: prospects for a broadly protective universal influenza vaccine. Nat Med 2010;16(12):1389-91). Figure 2 shows the phylogenic tree of the several hemagglutinin (H) subtypes. Group 1 may be further divided into three clusters, that is cluster H1-like (H1, H2, H5, H6), cluster H11-like (H11, H13 and H16) and cluster H9-like (H9, H8 and H12). Similarly, group 2 may be divided into other two clusters: cluster H3-like (H3, H4 and H14) and cluster H7-like (H7, H10 and H15).

On the basis of the broad neutralizing activity (also extended to the tested H3 isolates) demonstrated by IgG PN-SIA-28 and of the mutual phylogenic distances between the different subtypes, a neutralizing activity thereof is also likely towards isolates belonging to other subtypes. In particular, we can infer that:
1) The neutralizing activity is extremely likely to be extended to isolates belonging to all the cluster H1-like subtypes, that is H2, H5 and H6.
2) The neutralizing activity is very likely to be extended to isolates belonging to the cluster H9-like, that is H9, H8 or H12.
3) The neutralizing activity is likely to be extended to isolates belonging to the cluster H11-like subtypes, that is H11, H 13 or H12.
4) The activity towards subtypes belonging to group 2 is less easily predictable. In this connection, we can state that:
   a. The activity of PN-SIA28 is extremely likely to be extended to other isolates belonging to H3 subtype in addition to those already considered.
   b. It is possible that the activity of PN-SIA28 is extended to isolates belonging to other cluster H3-like subtypes, that is H4 and H 14.
   c. The activity of PN-SIA28 is less likely to be extended to isolates belonging to cluster H7-like subtypes, that is H7, H10 and H15.

Preliminary experiments were performed to verify these hypotheses, in order to assess the ability of IgG PN-SIA28 to bind recombinant proteins belonging to the subtypes hitherto isolated in the human species. Taking into consideration that the region recognized by PN-SIA28 is a highly neutralizing epitope, this finding allows to associate the ability of binding a certain subtype with the actual ability of neutralizing it.

In this context, it has been decided to proceed by applying an experimental approach already previously used by the present inventors, which comprises the synthesis of artificial genes encoding full-length hemagglutinins belonging to different subtypes, and the subsequent expression thereof on the surface of cells transfected with the individual constructs (Burioni R, Canducci F, Mancini N, et al. Monoclonal antibodies isolated from human B cells neutralize a broad range of H1 subtype influenza A viruses including swine-origin Influenza virus (S-OIV). Virology 2010;399(1):144-52; Burioni R, Canducci F, Mancini N, et al. Molecular cloning of the first human monoclonal antibodies neutralizing with high potency swine-origin influenza A pandemic virus (S-OIV). New Microbiol 2009;32(4):319-24). Thus, sequences of genes encoding HA from isolates belonging to subtypes that, in addition to H1 and H3, have already affected humans were selected from online databases (http://www.ncbi.nlm.nih.gov/nuccore; http://openflu.vital-it.ch/browse.php#results).

The following are the selected isolates:
- A/Ann Arbor/6/60 (H2N2)
- A/Vietnam/1203/2004 clade 1 (H5N1)
- LAIV A/chicken/Hong Kong/G9/97 (H9N2)
- A/New York/107/2003 (H7N2)

The transfected cells were then analyzed by immunofluorescence and FACS with PN-SIA28, and the results are summarized in Table 4. The speculations done on the basis of the phylogenic distances between the different subtypes were fully confirmed by the results obtained. Thus, this allows to believe that PN-SIA28's activity is at least extended to subtypes H2N2, H5N1 and H9N2.

**TABLE 4. Binding ability of PN-SIA28 towards cells transfected with hemagglutinins belonging to different subtypes.**

| **Phylogenic cluster** | **HA Subtype** | **Isolate** | **IF** | **FACS** |
|---|---|---|---|---|
| **H1-like** | H2 | A/Ann Arbor/6/60/(H2N2) | + | + |
| | H5 | A/Vietnam/1203/2004 clade 1 (H5N1) | + | + |
| **H9**-**like** | H9 | A/chicken/Hong Kong/G9/97 (H9N2) | + | + |
| **H7-like** | H7 | A/New York/107/2003 (H7N2) | - | - |

## Claims

1. A human monoclonal antibody specifically directed against the influenza A virus hemagglutinin antigen and capable of binding and neutralizing a plurality of influenza A virus subtypes including at least the H1 subtype and the H3 subtype, **characterized in that** it is a full-length IgG and further **characterized in that** it recognizes a conserved epitope located in the hemagglutinin stem region, said epitope comprising the amino acid residues His25, His45, Thr315 and Asn336 of the HA1 hemagglutinin subunit and the amino acid residues Thr358, Met360, Ile361 or Val361, Asp362, Gly363, Trp364, Yhr384, Thr392, Val395 and Glu400 of the HA2 hemagglutinin subunit, the numbering of said amino acid residues being based on the H1N1 hemagglutinin amino acid sequence designated as SEQ ID NO: 5.

2. The human monoclonal antibody according to claim 1, which is capable of binding and neutralizing a plurality of influenza A virus subtypes including at least the H1 subtype, the H3 subtype and the H5 subtype.

3. The human monoclonal antibody according to claim 1, which is capable of binding and neutralizing a plurality of influenza A virus subtypes including at least the H1 subtype, the H3 subtype and the H2 subtype.

4. The human monoclonal antibody according to claim 1, which is capable of binding and neutralizing a plurality of influenza A virus subtypes including at least the H1 subtype, the H3 subtype and the H9 subtype.

5. The human monoclonal antibody according to claim 1, which is capable of binding and neutralizing a plurality of influenza A virus subtypes including at least the H1 subtype, the H3 subtype, the H5 subtype and the H2 subtype.

6. The human monoclonal antibody according to claim 1, which is capable of binding and neutralizing a plurality of influenza A virus subtypes including at least the H1 subtype, the H3 subtype, the H5 subtype and the H9 subtype.

7. The human monoclonal antibody according to claim 1, which is capable of binding and neutralizing a plurality of influenza A virus subtypes including at least the H1 subtype, the H3 subtype, the H2 subtype and the H9 subtype.

8. The human monoclonal antibody according to claim 1, which is capable of binding and neutralizing a plurality of influenza A virus subtypes including at least the H1 subtype, the H3 subtype, the H5 subtype, the H2 subtype and the H9 subtype.

9. The human monoclonal antibody according to any of claims 1 to 8, comprising a heavy chain variable region comprising the amino acid sequence SEQ ID NO:1 and a light chain variable region comprising the amino acid sequence SEQ ID NO:2.

10. The human monoclonal antibody according to any of claims 1 to 9, wherein the heavy chain variable region is encoded by the nucleic acid sequence SEQ ID NO:3 and the light chain variable region is encoded by the nucleic acid sequence SEQ ID NO:4.

11. A pharmaceutical composition comprising an effective amount of at least an antibody according to any of claims 1 to 10 and a pharmaceutically acceptable vehicle and/or diluent.

12. The human monoclonal antibody according to any of claims 1 to 10, for use in a therapeutic or prophylactic medicament against a pathology which is directly or indirectly caused by influenza A virus infection.

13. The human monoclonal antibody for use according to claim 12, wherein the pathology caused by influenza A virus infection is the influenza syndrome.

14. An assay method for detecting, in a biological sample from a patient, the presence of anti-influenza virus antibodies having heterosubtype cross-neutralizing activity, comprising the step of contacting said biological sample with an antibody according to any of claims 1 to 10 as a specific assay reagent.

15. A diagnostic kit comprising as a specific reagent an antibody according to any of claims 1 to 10, said kit being intended in particular for use in a method for detecting or quantifying, in a biological sample from a patient, anti-influenza A virus antibodies having heterosubtype cross-neutralizing activity.

16. An assay method for detecting, in an immunogenic or vaccine composition, the presence of influenza A virus epitopes capable of eliciting, in a subject to which it is administered, anti-influenza A virus antibodies having heterosubtype cross-neutralizing activity towards the influenza A virus, comprising the step of contacting said composition with an antibody according to any of claims 1 to 10 as a specific assay reagent.

17. A mimotope specifically directed against the idiotype of an antibody according to any of claims 1 to 10.

## Patentansprüche

1. Menschlicher monoklonaler Antikörper, der spezifisch gegen das Influenza A-Virus-Hämagglutinin-Antigen gerichtet ist und eine Mehrzahl von Influenza A-Virussubtypen, einschließlich mindestens den H1-Subtyp und den H3-Subtyp, binden und neutralisieren kann, **dadurch gekennzeichnet, dass** er ein Volllängen-IgG ist und ferner **dadurch gekennzeichnet, dass** er ein konserviertes Epitop erkennt, das in der Hämagglutinin-Stammregion lokalisiert ist, wobei das Epitop die Aminosäurereste His25, His45, Thr315 und Asn336 der HA1-Hämagglutinin-Untereinheit und die Aminosäurereste Thr358, Met360, Ile361 oder Val361, Asp362, Gly363, Trp364, Yhr384, Thr392, Val395 und Glu400 der HA2-Hämagglutinin-Untereinheit umfasst, wobei die Nummerierung der Aminosäurereste auf der H1N1-Hämagglutinin-Aminosäuresequenz beruht, die als SEQ ID NO:5 bezeichnet ist.

2. Menschlicher monoklonaler Antikörper nach Anspruch 1, der eine Mehrzahl von Influenza A-Virussubtypen, einschließlich mindestens den H1-Subtyp, den H3-Subtyp und den H5-Subtyp, binden und neutralisieren kann.

3. Menschlicher monoklonaler Antikörper nach Anspruch 1, der eine Mehrzahl von Influenza A-Virussubtypen, einschließlich mindestens den H1-Subtyp, den H3-Subtyp und den H2-Subtyp, binden und neutralisieren kann.

4. Menschlicher monoklonaler Antikörper nach Anspruch 1, der eine Mehrzahl von Influenza A-Virussubtypen, einschließlich mindestens den H1-Subtyp, den H3-Subtyp und den H9-Subtyp, binden und neutralisieren kann.

5. Menschlicher monoklonaler Antikörper nach Anspruch 1, der eine Mehrzahl von Influenza A-Virussubtypen, einschließlich mindestens den H1-Subtyp, den H3-Subtyp, den H5-Subtyp und den H2-Subtyp, binden und neutralisieren kann.

6. Menschlicher monoklonaler Antikörper nach Anspruch 1, der eine Mehrzahl von Influenza A-Virussubtypen, einschließlich mindestens den H1-Subtyp, den H3-Subtyp, den H5-Subtyp und den H9-Subtyp, binden und neutralisieren kann.

7. Menschlicher monoklonaler Antikörper nach Anspruch 1, der eine Mehrzahl von Influenza A-Virussubtypen, einschließlich mindestens den H1-Subtyp, den H3-Subtyp, den H2-Subtyp und den H9-Subtyp, binden und neutralisieren kann.

8. Menschlicher monoklonaler Antikörper nach Anspruch 1, der eine Mehrzahl von Influenza A-Virussubtypen, einschließlich mindestens den H1-Subtyp, den H3-Subtyp, den H5-Subtyp, den H2-Subtyp und den H9-Subtyp, binden und neutralisieren kann.

9. Menschlicher monoklonaler Antikörper nach einem der Ansprüche 1 bis 8, der einen variablen Bereich der schweren Kette, der die Aminosäuresequenz SEQ ID NO:1 umfasst, und einen variablen Bereich der leichten Kette umfasst, der die Aminosäuresequenz SEQ ID NO:2 umfasst.

10. Menschlicher monoklonaler Antikörper nach einem der Ansprüche 1 bis 9, bei dem der variable Bereich der schweren Kette durch die Nukleinsäuresequenz SEQ ID NO:3 kodiert ist und der variable Bereich der leichten Kette durch die Nukleinsäuresequenz SEQ ID NO:4 kodiert ist.

11. Pharmazeutische Zusammensetzung, die eine wirksame Menge mindestens eines Antikörpers nach einem der Ansprüche 1 bis 10 und einen pharmazeutisch verträglichen Träger und/oder ein pharmazeutisch verträgliches Verdünnungsmittel umfasst.

12. Menschlicher monoklonaler Antikörper nach einem der Ansprüche 1 bis 10 zur Verwendung in einem therapeutischen oder prophylaktischen Medikament gegen eine Krankheit, die direkt oder indirekt durch eine Influenza A-Virusinfektion verursacht wird.

13. Menschlicher monoklonaler Antikörper zur Verwendung nach Anspruch 12, wobei die Krankheit, die durch eine Influenza A-Virusinfektion verursacht wird, das Influenza-Syndrom ist.

14. Testsystem zum Erfassen der Gegenwart von Anti-Influenza-Virus-Antikörpern mit einer Heterosubtyp-Kreuzneutralisierungsaktivität in einer biologischen Probe von einem Patienten, umfassend den Schritt des Inkontaktbringens der biologischen Probe mit einem Antikörper nach einem der Ansprüche 1 bis 10 als ein spezifisches Testreagenz.

15. Diagnostisches Kit, das als spezifisches Reagenz einen Antikörper nach einem der Ansprüche 1 bis 10 umfasst, wobei das Kit insbesondere zur Verwendung in einem Verfahren zum Erfassen oder Quantifizieren von Anti-Influenza-Virus-Antikörpern mit einer Heterosubtyp-Kreuzneutralisierungsaktivität in einer biologischen Probe von einem Patienten vorgesehen ist.

16. Testverfahren zum Erfassen, in einer immunogenen Zusammensetzung oder einer Impfstoffzusammensetzung, der Gegenwart von Influenza A-Virusepitopen, die in einem Lebewesen, an das sie verabreicht wird, Anti-Influenza A-Virusantikörper mit einer Heterosubtyp-Kreuzneutralisierungsaktivität in Bezug auf das Influenza A-Virus auslösen können, umfassend den Schritt des Inkontaktbringens der Zusammensetzung mit einem Antikörper nach einem der Ansprüche 1 bis 10 als spezifisches Testreagenz.

17. Mimotop, das spezifisch gegen den Idiotyp eines Antikörpers nach einem der Ansprüch 1 bis 10 gerichtet ist.

## Revendications

1. Anticorps monoclonal humain dirigé spécifiquement contre l'antigène hémagglutinine du virus de la grippe A et capable de se lier et de neutraliser une pluralité de sous-types de virus de la grippe A comprenant au moins le sous-type H1 et le sous-type H3, **caractérisé en ce qu'**il est une IgG de pleine longueur et **caractérisé en outre en ce qu'**il reconnaît un épitope conservé situé dans la région souche de l'hémagglutinine, ledit épitope comprenant les résidus d'acide aminé His25, His45, Thr315 et Asn336 de la sous-unité HA1 de l'hémagglutinine et les résidus d'acide aminé Thr358, Met360, Ile361 ou Val361, Asp362, Gly363, Trp364, Yhr384, Thr392, Val395 et Glu400 de la sous-unité HA2 de l'hémagglutinine, la numérotation desdits résidus d'acide aminé étant basée sur la séquence d'acides aminés de l'hémagglutinine H1N1 représentée par SEQ ID NO : 5.

2. Anticorps monoclonal humain selon la revendication 1, qui est capable de se lier et de neutraliser une pluralité de sous-types de virus de la grippe A comprenant au moins le sous-type H1 le sous-type H3 et le sous-type H5.

3. Anticorps monoclonal humain selon la revendication 1, qui est capable de se lier et de neutraliser une pluralité de sous-types de virus de la grippe A comprenant au moins le sous-type H1 le sous-type H3 et le sous-type H2.

4. Anticorps monoclonal humain selon la revendication 1, qui est capable de se lier et de neutraliser une pluralité de sous-types de virus de la grippe A comprenant au moins le sous-type H1 le sous-type H3 et le sous-type H9.

5. Anticorps monoclonal humain selon la revendication 1, qui est capable de se lier et de neutraliser une pluralité de sous-types de virus de la grippe A comprenant au moins le sous-type H1 le sous-type H3, le sous-type H5 et le sous-type H2.

6. Anticorps monoclonal humain selon la revendication 1, qui est capable de se lier et de neutraliser une pluralité de sous-types de virus de la grippe A comprenant au moins le sous-type H1 le sous-type H3, le sous-type H5 et le sous-type H9.

7. Anticorps monoclonal humain selon la revendication 1, qui est capable de se lier et de neutraliser une pluralité de sous-types de virus de la grippe A comprenant au moins le sous-type H1, le sous-type H3, le sous-type H2 et le sous-type H9.

8. Anticorps monoclonal humain selon la revendication 1, qui est capable de se lier et de neutraliser une pluralité de sous-types de virus de la grippe A comprenant au moins le sous-type H1, le sous-type H3, le sous-type H5, le sous-type H2 et le sous-type H9.

9. Anticorps monoclonal humain selon l'une quelconque des revendications 1 à 8, comprenant une région variable de chaîne lourde comprenant la séquence d'acides aminés SEQ ID NO : 1 et une région variable de chaîne légère comprenant la séquence d'acides aminés SEQ ID NO : 2.

10. Anticorps monoclonal humain selon l'une quelconque des revendications 1 à 9, dans lequel la région variable de chaîne lourde est codée par la séquence d'acide nucléique SEQ ID NO : 3 et la région variables de chaîne légère est codée par la séquence d'acide nucléique SEQ ID NO : 4.

11. Composition pharmaceutique comprenant une quantité efficace d'au moins un anticorps selon l'une quelconque des revendications 1 à 10 et un support et/ou diluant pharmaceutiquement acceptables.

12. Anticorps monoclonal humain selon l'une quelconque des revendications 1 à 10, pour son utilisation dans un médicament thérapeutique ou prophylactique contre une pathologie qui est directement ou indirectement provoquée par une infection liée au virus de la grippe A.

13. Anticorps monoclonal humain pour son utilisation selon la revendication 12, dans lequel la pathologie provoquée par une infection liée au virus de la grippe A est le syndrome grippal.

14. Procédé d'analyse permettant de détecter, dans un échantillon biologique provenant d'un patient, la présence d'anticorps anti-virus de la grippe possédant une activité de neutralisation croisée d'hétérosous-type, comprenant l'étape de mise en contact dudit échantillon biologique avec un anticorps selon l'une quelconque des revendications 1 à 10 en tant que réactif d'analyse spécifique.

15. Kit de diagnostic comprenant, en tant que réactif spécifique, un anticorps selon l'une quelconque des revendications 1 à 10, ledit kit étant en particulier destiné à être utilisé dans un procédé de détection ou de quantification, dans un échantillon biologique provenant d'un patient, des anticorps anti-virus de la grippe A possédant une activité de neutralisation croisée d'hétérosous-type.

16. Procédé d'analyse permettant de détecter, dans une composition immunogène ou vaccinale, la présence d'épitopes de virus de la grippe A capables d'induire, chez un sujet auquel elle est administrée, des anticorps anti-virus de la grippe A possédant une activité de neutralisation croisée d'hétérosous-type dirigée contre le virus de la grippe A, comprenant l'étape de mise en contact de ladite composition avec un anticorps selon l'une quelconque des revendications 1 à 10 en tant que réactif d'essai spécifique.

17. Mimotope dirigé spécifiquement contre l'idiotype d'un anticorps selon l'une quelconque des revendications 1 à 10.
